(19) Europäisches Patentamt
European Patent Office
Office européen des brevets

(11) **EP 3 960 200 A1**

(12) **EUROPEAN PATENT APPLICATION**

(43) Date of publication:
**02.03.2022 Bulletin 2022/09**

(21) Application number: **20192433.9**

(22) Date of filing: **24.08.2020**

(51) International Patent Classification (IPC):
**A61K 41/00** (2020.01)  **A61P 35/00** (2006.01)

(52) Cooperative Patent Classification (CPC):
**A61K 41/0052; A61P 35/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Politechnika Wroclawska
50-370 Wrocklaw (PL)**

(72) Inventors:
• **Bauer, Joanna
51-361 Wilczyce (PL)**
• **Devappa Thorat, Nanasaheb
415102 Satara (IN)**

(74) Representative: **Pawlowska, Justyna
Kancelaria Patentowa Justyna Pawlowska
Wzgorze Bernardowo 263B/6
81-583 Gdynia (PL)**

(54) **ACTIVE MAGNETIC NANOPARTICLES, METHOD OF OBTAINING MAGNETIC NANOPARTICLES, MEDICAL AND DIAGNOSTIC USE OF MAGNETIC NANOPARTICLES UND USE AS A CARRIER OF BIOLOGICALLY ACTIVE AGENTS AND DRUGS, ESPECIALLY IN TUMORS TREATMENT, ESPECIALLY IN CANCERS INCLUDED BREAST CANCER**

(57)  Biologically active nanoparticles with a magnetic core and a gold coating characterized according to the invention that they have magnetic and plasmonic properties activated by an alternating magnetic field and/or infrared light, in a form of core-coating where magnetic core is $Fe_3O_4$, while at least one coating is gold made. In embodiment nanoparticle contains a biologically active agent especially for treatment of tumour s cells.

EP 3 960 200 A1

**Description**

The invention relates to magnetic nanoparticles activated by infrared light and/or magnetic field, the nanoparticles are with one or more metallic coating/layer deposited on the surface of a magnetic core (core) with supermagnetic and plasmonic properties. The invention further relates to the medical and diagnostic use of nanoparticles. In an embodiment, the nanoparticles additionally contain a biologically active agent and/or a drug deposited on the core-shell structure, in particular targeting cancer treatment agent. In this embodiment, the use additionally relates to carriers of biologically active agents/substances and/or an agent that has a therapeutic effect per se, in particular for use as an anti-tumor agent.

There have been significant advancements in the treatment of breast cancer in recent years but the mortality rate of the patients remains high. Surgery, radiation and chemotherapies are still main pillars of cancer treatments. All these therapies have major disadvantage, such as lack of specificity, adverse toxic effects on normal organs and tissues, poor bioavailability of chemotherapeutic drugs, and developed in patients a resistance to the treatment. In view of the severe and widespread morbidity associated with breast cancer in women and high mortality, there is serious unmet clinical need for new therapeutic clinical approaches for, effective targeted therapy with possibility of simultaneous monitoring to tackle the tumour - cancer in advanced clinical stage. Some of earlier inventions report the non-conventional cancer therapeutic approaches based on nanomedicine. For example, invention was disclosed inUS2018/0280546A1related to stimuli responsive magnetic and plasmonic nanoparticles co-loaded with a therapeutic agent encapsulated within liposomes that is used to deliver drugs into tumor cells and tumor imaging, However, the study demonstrated only on the drug delivery and did not discuss about the new generation cancer therapy approach. Similarly, nano-systems comprising dendrimers and phthalocyanine for photodynamic therapy and endovascular fluorescence-based imaging of inflammatory lesion, or a tumor tissues was disclosed in WO2017/191219. In WO 2016/073348 therapeutic delivery of drugs using the paramagnetic nanoparticle (PMNP) for inducing vascular inflammation was disclosed. The invention reports a diagnostic kit that comprises the PMNP enhanced delivery of the therapeutic agent that induces a significant enhancement of vascular leakiness in a human body. The increased vascular leakiness allows for enhanced local delivery of therapeutics and imaging agents for diagnostic purposes.
In WO 2015/023653 methods, systems, and devices for performing photothermal therapy to treat cancer using magnetic nanoparticles with a biocompatible coating surrounding a highly crystallized magnetic $Fe_3O_4$ core was disclosed.

There is still need for providing of new therapeutical treatment method for tumours/cancers treatment that is additionally improved by further therapeutic method, especially breast cancer.

Multifunctional hybrid nanostructures (HNCs) with organic-inorganic-design appear to be a promising route to meet the current therapeutics needs required for efficient tumours treatment. Nanomedicine has emerged as a rapidly developing field to face the current diagnostics and therapeutics challenges. HNCs with combined magnetic/plasmonic characters are of particular interest in tumour/cancer nanomedicine. The inventors of the applied inventionspecial designed magnetic and plasmonic new nanoparticle - HNC with double functionality (magnetic and plasmonic) in order to provide the particle/medium for use in in therapy/treatment of cancers/tumors, and simultaneously use in diagnostics - as agent/carrier/medium for use in diagnostic imaging study - computer tomography / magnetic resonance imaging / Raman spectroscopy in the field of cancer nanomedicine. The goal of the invention was to provide active nanoparticle in a form of core-shell (layer) with double characteristics/properties in one particle - magnetic and plasmonic in order to accumulate different characteristics and uses in diagnostic and in therapy at once. The invention give benefits that designed HNC has better properties in terms of efficacy for diagnostics and therapy/treatment in comparison to known magnetic-plasmonic agents of . Functionalizing of the well-developed superparamagnetic iron oxides with gold, and in embodiment of the invention biological active compound and therapeutical/treatment compound - drug, in order to build core-shell nanocarriers, enables magnetically guided enrichment of therapeutic carrier at tumour site, as well as enables performance of magnetic resonance imaging (MRI) eg. $T_2$-weighted one and/or other imaging - computer tomography (TK) and Raman spectrometry allowing to find localisation of tumour in whole-body - tumor delineation with high spatial resolution. Addition of therapeutic compound - drug into the designed nanoparticles improve/enhance the effects. Simultaneously, the intense light absorption by gold shell in near-infrared (NIR) region makes such nanocarriers promising light activated agents to produce highly localized heat for selective cancer photothermal therapy (PTT). On the other hand under the influence of magnetic field - magnetic hyperthermia MHT is provoked/created. The conjugation of the core shell nanocarriers with tumour - cancer cell targeting compound - moiety - that recognizes nucleoli of the cell or receptors overexpressed on the surface of a tumour cell will enhance specific tumor cell uptake and increase the possibility of transporting particles to an intended target site such as tumor.

In the embodiment, the goal was to improve - intensify effect of innovation beyond the known in the art non-conventional tumour/cancer therapy approaches such as PTT and MHT, and provision of synergetic effect of these therapies/treatment by using single nanoplatform with simultaneously provided drug therapy and in one of the embodiment with agent recognized tumour cells. PTT and MHT is an emerging cancer therapies, which in principle requires three interacting elements: (i) a light-magnetic activatable materials, the so-called nanoparticles; (ii) light of appropriate wavelengths; and (iii) suitable magnetic field of strength. Invention involved use of two treatment modalities simultaneously (PTT+MHT) to synergistically treat tumor cells in patients with using one type of nanoparticle, using biologically active agent for selective administration of nanoparticles within targeted tumour, using targeted drug and imaging techniques for tumour visualization. Firstly, an administration of affective dosage of provided photo-magnetic nanoparticles, capable of self-concentrating in tumour (finding localization of tumour), with drug to tumour localisation, taken place. Then, exposition of patient to photon and magnetic stimulation and additionally for imaging diagnostics by imaging tool. The synergistic therapeutic combination provides a rapid, safe, and effective treatment of local tumor tissues, better than each modality alone.

Currently available HNCs are capable of releasing therapeutic agents inside tumor when subject to internal stimulus such as temperature, pH, redox potential, and enzymes what disclosed in:

Espinosa A, Di Corato R, Kolosnjaj-Tabi J, Flaud P, Pellegrino T, Wilhelm C. Duality of Iron Oxide Nanoparticles in Cancer Therapy: Amplification of Heating Efficiency by Magnetic Hyperthermia and Photothermal Bimodal Treatment. ACS Nano 2016;10:2436-46. https://doi.org/10.1021/acsnano.5b07249.

Huang L, Ao L, Hu D, Wang W, Sheng Z, Su W. Magneto-Plasmonic Nanocapsules for Multimodal-Imaging and Magnetically Guided Combination Cancer Therapy. Chem Mater 2016;28:5896-904. https://doi.org/10.1021/acs.chemmater.6b02413.

Espinosa A, Bugnet M, Radtke G, Neveu S, Botton GA, Wilhelm C, et al. Can magneto-plasmonic nanohybrids efficiently combine photothermia with magnetic hyperthermia? Nanoscale 2015;7:18872-7. https://doi.org/10.1039/c5nr06168g.

Tseng Y-J, Chou S-W, Shyue J-J, Lin S-Y, Hsiao J-K, Chou P-T. A Versatile Theranostic Delivery Platform Integrating Magnetic Resonance Imaging/Computed Tomography, pH/ cis -Diol Controlled Release, and Targeted Therapy. ACS Nano 2016;10:5809-22. https://doi.org/10.1021/acsnano.5b08130.

Thorat ND, Bohara RA, Noor MR, Dhamecha D, Soulimane T, Tofail SAM. Effective Cancer Theranostics with Polymer Encapsulated Superparamagnetic Nanoparticles: Combined Effects of Magnetic Hyperthermia and Controlled Drug Release. ACS Biomater Sci Eng 2017;3:1332-40. https://doi.org/10.1021/acsbiomaterials.6b00420.

Such a pathological change or in vivo stimulation can be executed in a static passive mode and faces challenges of chemotherapeutic dosage control, timing, and duration of the therapeutic drug release. Additionally, the in vivo stimulation approach would not allow for self-regulated controlled chemotherapeutic drug release.

In the treatment of tumour/cancer, prior drug release kinetics and drug dosing are difficult to predict. To achieve high therapeutic potential, drug dosing will need to be actively managed. As a result, there remains a need for novel and noninvasive nanosystem technology that can be activated remotely in targeted manner. Aline, the goal was to provide novel approach of remotely triggered systems that enable better clinical results over in vivo triggers. The use of externally applied stimuli in the form of physical forces along with administered signaling HNCs could allow for drug release at the right time and at the right dose needed for a cancer/tumou patient.

The present invention relates to core shell photo-magnetic nanoparticle based novel formulation by combining gold shell (Au) on magnetic core ($Fe_3O_4$). The nanoparticles/nanosystem have magnetic and plasmotic properties at once. In embodiment, nanoparticles are link/bounded with bi-functional anticancer aptamer (AS1411) and/or dug, specially cytostatic drug. Aptamer allows for administration of nanoparticles, and in embodiment drug and/or nucleic acids, into targeted tumour cells, especially breast cancer cells. The nanocarriers of the invention are of particular utility as theranostic (therapeutic and diagnostic and therapy monitoring) nano-systems. The nano-systems may be used in, without limitation, near infrared (NIR) light activated photothermal therapy (PTT) and/or magnetic fluid hyperthermia (MFH) therapy and/or magnetic resonance imaging (MRI) follow-up therapy monitoring, in particular, cancer diseases. The nanosystems may also be of utility in relation to other diseases.

The invention enables synergistic therapy, especially anti-tumour therapy - it enables simultaneous magnetic hyperthermia (MHT) and photothermal therapy (PTT) to be performed. Nanoparticles allow to use of two therapeutic techniques - methods - for the synergistic treatment/therapy especially in the case of primary cancer cells in patient. The first element of the method involves magnetic hyperthermia (MHT) using photomagnetic molecules activate in an alternating magnetic field. The second element of method involves photomagnetic exposure of the activated molecules to near-infrared photon radiation in order to selective photothermal therapy (PTT). In an embodiment, by the mentioned light and magnetic techniques, the activation of nanoparticle also enables for simultaneouse release of the therapeutic/treatment and/or biologically active agent. In embodiment, an aptamer is used as biologically active agent. Aptamer recognizes tomour cells on the basis of biological affinity, i.e. it causes that the nanoparticles bind only to tumour cells and not to healthy tissues, and then application of the magnetic field and NIR together allow for controlled drug release and MHT and PTT therapy. It releases from nanoparticle occurs under the influence of an alternating magnetic field and/or infrared light - NIR radiation. In a particular embodiment, the invention additionally enables the use of nanoparticle-molecule for the administration of an antitumour substance/agent and simultaneous induction of MHT magnetic hyperthermia and/or PTT thermal therapy, in particular the targeted administration of drug, by means of an aptamer, especially cytotoxic drug to tumor cells, including breast cancer. The core of the nanoparticle - Fe3O4 is supermagnetic and particles subjected to an alternating magnetic field cause magnetic hyperthermia, i.e. overheating of a tumor, e.g. a tumor, while the gold coat has plasmonic properties, i.e. under the influence of NIR light, optical energy is converted into heating/thermal one, i.e. the so-called photothermal effect is generated, which also causes overheating of tumor tissue. Such a synergistic combination provides quick, safe and effective treatment of local and distant tumors, moreover the obtained effect is better than if each technique was used separately. Furthermore, due to the specific structure of the nanoparticles, the invention covers the diagnostic use of the nanoparticle as a means for visualization in magnetic resonance and/or computed tomography and/or Raman spectroscopy. The Fe3O4 nanoparticle core is supermagnetic and thus increases the visibility of nanoparticles in MRI, and the gold coat improves the visibility of nanoparticles in computed tomography and Raman spectroscopy. Thus, administration of targeted nanoparticles results in a better visualization of the cancerous cells (primary and metastatic tumors).

The present invention provides methods, systems, and devices for performing a synergistic therapy with use of two combined treatment modalities (MHT + PTT), therapeutically effective amount of an antitumour treatment/cancer chemotherapeutic agent (eg. doxorubicin (DOX) and biologically active agent - aptamer (AS1411) in order to treat primary tumour/cancer cells and imaging diagnostic method for tumour diagnosis, in accordance with a embodiments of the presently disclosed subject matter.

In one embodiment, the invention provided materials and methods for synthesizing nanoparticles (carrier, nanosystem), particularly, photo-magnetic nanoparticles and methods for using them in thumour/cancer theranostics.

Synthesizing Iron (II, III) oxide ($Fe_3O_4$) core and gold shell/layer (Au) nanoparticles takes placed into three step low temperature chemical reflux method with the use of a condensate reflux condenser. In a particular embodiment the chemical reflux method comprises $Fe_3O_4$ nanoparticles as seeds and reducing $Au^{+3}$ using a reducing agent such as, oleylamine, Cetyl Trimethyl Ammonium Bromide (CTAB) and sodium citrate.

Certain embodiments of the invention provide a formulation comprising photo-magnetic $Fe_3O_4$@Au nanoformulations bund to or co loaded with chemotherapeutic agents and biologically active agent, such as doxorubicin and aptamer AS 1411.

Photo-magnetic nanocarriers synthesized with the method described herein have superparamagnetic and optical properties advantageous for theranostic applications, particularly, for magnetic fluid hyperthermia (MHT), phothermal therapy (PTT), magnetically guided transport into cancer cells.

In some embodiments, the magnetic fluid hyperthermia therapy is conducted using a device induction heating unit in the applied frequency 265 kHz and applied magnetic field ranging from 83.8 to 502.8 Oe.

In some embodiments, the photothermal therapy is conducted with a near infrared (NIR) laser or other NIR emitting device configured to emit electromagnetic radiation in the wavelengths between about 650 nm and 1000 nm, and power ranging from 100 to 500 mW.

In particular embodiments, the present invention provides methods of treating breast cancer cells in a subject comprising: (i) administering to the subject a formulation comprising photo-magnetic nanocarriers bound to, or co-loaded with, a chemotherapeutic agent and applying to the subject magnetic forces to release the chemotherapeutic cargo from $Fe_3O_4$@Au into breast cancer (MCF7) cells. The chemotherapeutic agent can be released into the cancer cells by applying an alternating magnetic field (AMF) and/or a near-infrared light (NIR) to the subject, the required energy source depending upon the nature of the nanocarriers.

A further embodiment of the invention provides a method for magnetic resonance imaging (MRI) and/or computer tomography and/or Raman spectroscopy for tumour cells visualisation in patient, via administration into patient, fix additionally with therapeutical drug in the particular embodiment, and/or allowing targeted to tumour cells (aptamer).

It is disclosed biodistribution of photo-magnetic nanoparticle/carriers in the major organs of animal (laboratory mice).

Multifunctional hybrid nanostructures (HNCs) appear to be a promising mediator to meet the modern therapeutic and diagnostic needs required for efficient cancer theranostics. The modern cancer theranostics demand the remote tumor targeting and activation of HNCs by the external physical stimuli, thus precluding harmful effects on healthy bystander cells. Moreover, advanced cancer nanomedicine relies on multifunctionality of nanostructures that can incorporate several functions within a single nanostructure, to offer a synergic proficiency by merging multiple therapeutic modalities.

The HNCs with combined magnetic/plasmonic characters are of particular interest in next generation personalized cancer medicine, such as tumor diagnosis with multiple imaging modalities and combination of therapeutic strategies for synergetic effects. One promising remotely activated synergetic cancer nanotherapy is based on the HNCs ability to generate heat under an external magnetic/light stimulus. Currently, iron oxide (magnetic) and gold (plasmonic) nanostructures are the best hybrid mediators for the development of synergetic MHT together with PTT. Localized plasmonic excitation by an infrared laser and magnetic field actuation can causes localized therapeutic effect and drug release that can destroy the tumor tissues without affecting the surrounding healthy environment.

The present innovation provided demonstration of photo-magnetic active HNCs for, cancer targeting, and controlled release of therapeutic drug/agents under magnetic and light stimulation to proof its theranostics capabilities.

In the description there are synonims such as plasmonic-magnetic nanoparticles/ nanocarriers/nanocompund/nanosystem/ $Fe_3O_4$@Au core shell nanostructure.

The invention was disclosed in examples and drawings, wherein the following is presented:

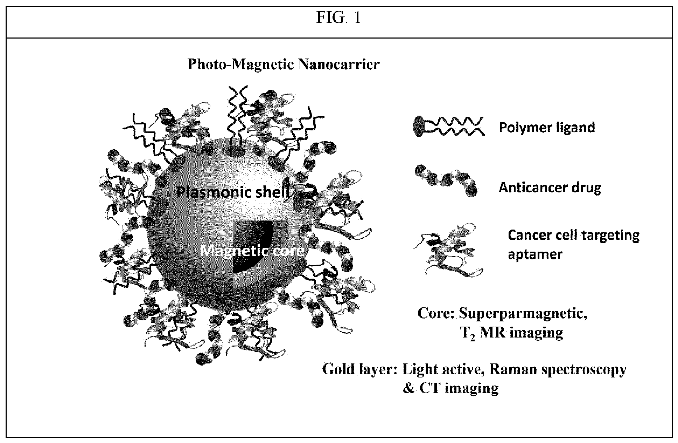

FIG. 1. Scheme of the proposed photomagnetic nanocarriers. Photo-magnetic nanocarriers are multifunctional and responsive for both, magnetic and light stimuli and contain the therapeutic, tumor targeting and imaging agents for cancer theranostics. The core shell structure of photo-magnetic nanocarriers (with magnetic core and plasmonic shell) have multimodal imaging abilities including MRI and CT which enables imaging guided cancer theranostics.

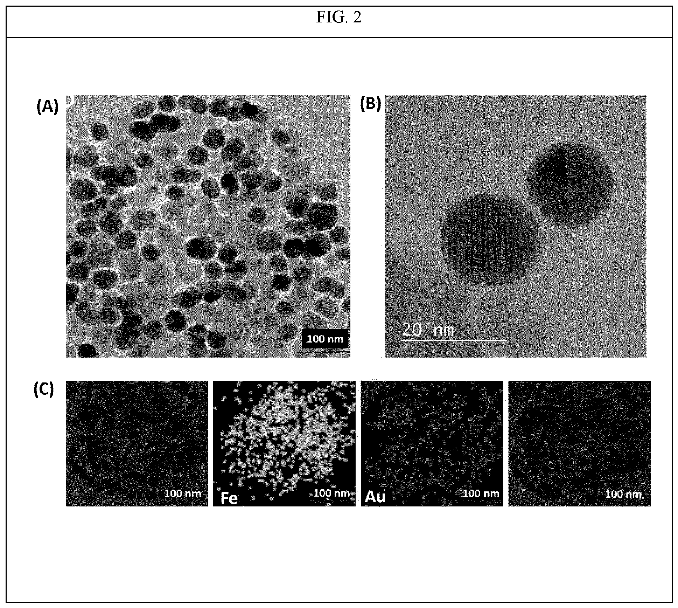

FIGS. 2A-2C. TEM image of $Fe_3O_4$@Au nanoparticles. [2A-B] TEM images of $Fe_3O_4$@Au nanoparticles synthesized by low temperature reflux method. The average diameter of $Fe_3O_4$@Au was 15 nm. [2C] TEM images showing the elemental structure of $Fe_3O_4$@Au nanoparticles, in order: core shell structure, iron (Fe), gold (Au) and fused images of all elements.

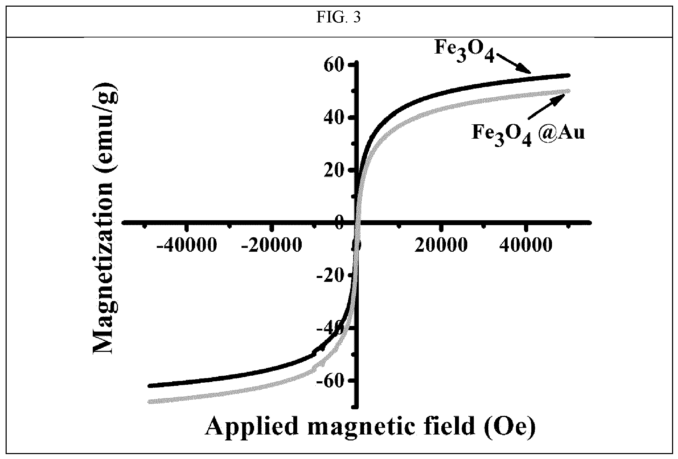

FIG. 3. Vibrating sample magnetometer (VSM) measurements, showing dependence between the applied magnetic field and the magnetization, for bare $Fe_3O_4$ and $Fe_3O_4$@Au nanoparticles. As shown, the $Fe_3O_4$@Au nanoparticles have superparamagnetic nature and very low coercively value.

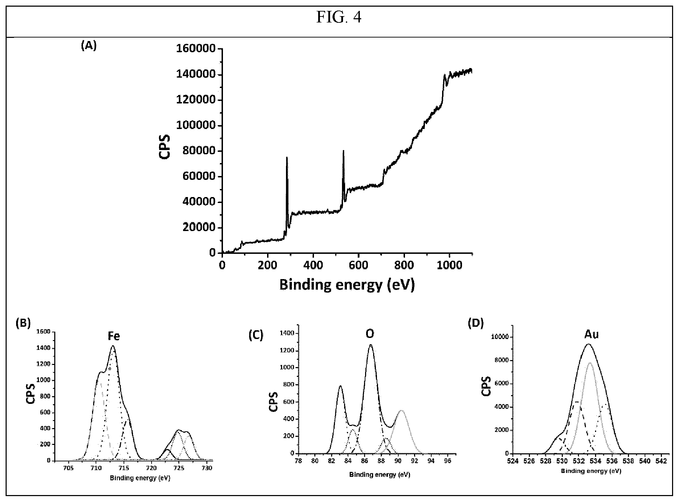

FIG. 4A-D. Full X-ray photoelectron spectra (XPS) of $Fe_3O_4$@Au nanoparticles showing counts per second (CPS) as a function of binding energy . XPS analysis confirmed the presence of Fe, Au, O, in the $Fe_3O_4$@Au core shell structure. [A] Full XPS spectrum of $Fe_3O_4$@Au nanoparticles. [B]. XPS spectrum for iron Fe. Fe exhibits peaks at 710 and 723 eV from $Fe_3O_4$. [C] XPS spectrum for oxygen O. $O_{1s}$ peaks are assigned at 528,6 eV, 531,9 eV, 534,5 eV. [D] XPS spectrum for gold Au. The presence of $Au_{4f7/2}$ & $Au_{4f5/2}$ is confirmed by the peaks at 86.4 eV and 88,3 eV.

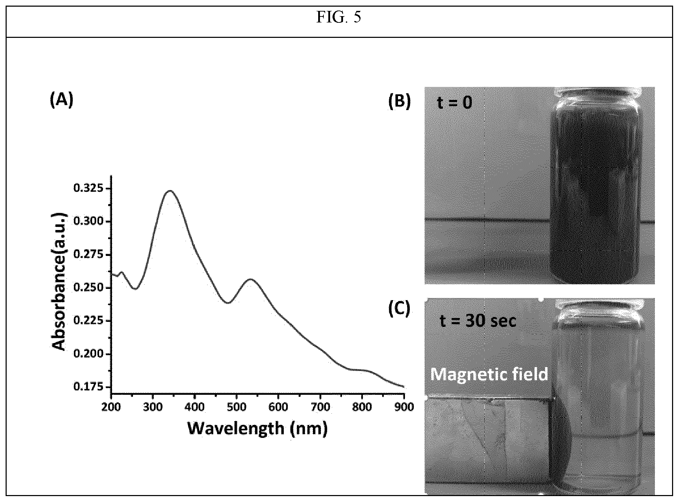

FIG. 5. [A] Absorbance spectrum of $Fe_3O_4$@Au nanoparticles in water. Pictures of $Fe_3O_4$@Au nanoparticles samples suspended in water without [B] and with applied magnetic field [C].

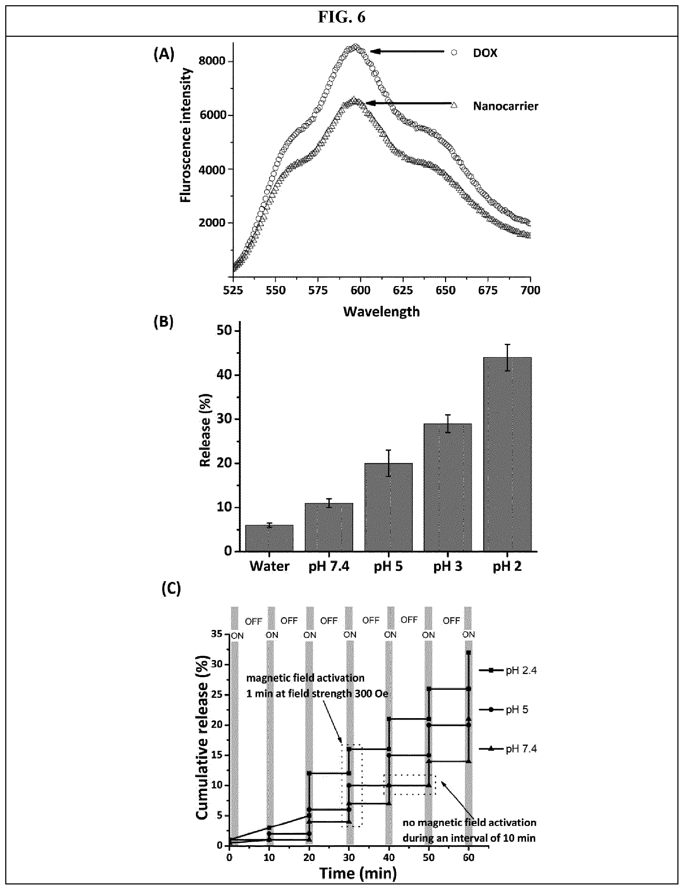

FIG. 6. Doxorubicin (DOX) loading on nanoparticles $Fe_3O_4$@Au and release in water. [A] Fluorescence spectra of DOX and photo-magnetic nanocarriers. (DOX: Ex. 480 nm, Em. 590 nm.) [B] DOX release studies from nanocarriers in water at different pH values after 24 h. [C] Stimuli-responsive doxorubicin DOX release from nanocarriers in woater in presence of alternating magnetic field at different pH. The AC magnetic field with strength of 20 kAm$^{-1}$ ≈ 300 Oe, and frequency f= 265 kHz was switched between "ON" and "OFF" modes. The counterparts without being simulated by magnetic field were used as a control.

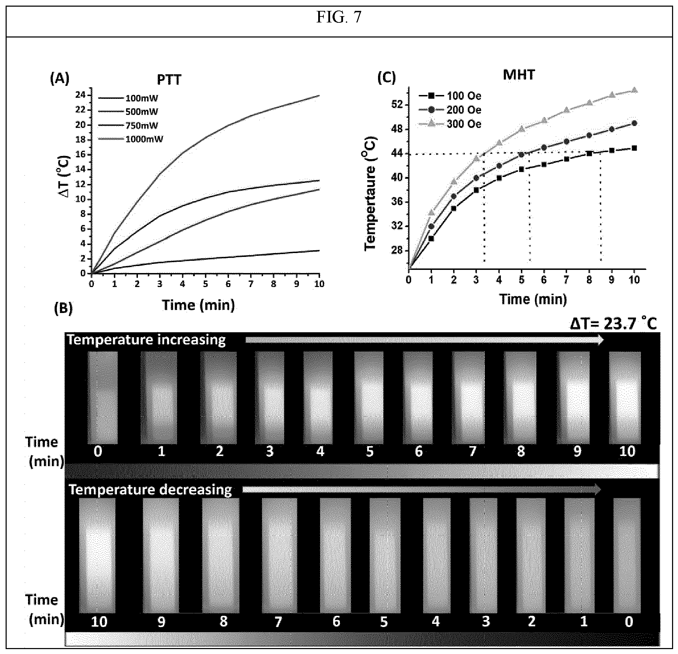

FIG. 7. Photo-magnetic nanocarriers can be stimulated by f both, light and magnetic field and thus may be applied for photothermal therapy, magnetic hyperthermia and combined therapy using both these modalities. [A] Photothermal effect of core shell $Fe_3O_4$@Au nanocarriers in water at different laser power values. [B] Thermal images of water suspension of photo-magnetic nanocarriers stimulated by NIR laser 808 nm with power 1W (0-10 - images of nanocarries suspension during laser stimulation, 10-0 - images of nanocarries suspension after the end of laser stimulation). [C] Magnetic fluid hyperthermia studies of $Fe_3O_4$@Au nanocarriers in water at different magnetic field strengths and constant frequency of 265 kHz. The samples were heated for 10 min. The temperature was measured using an optical fiber probe with an accuracy 0.1 °C.

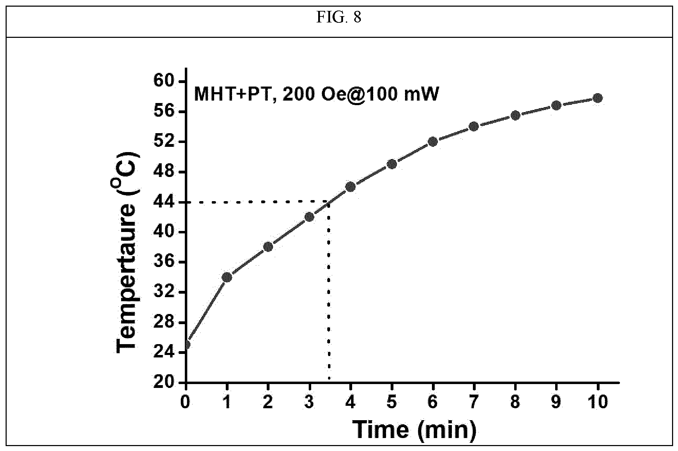

FIG. 8. Temperature rise of photomagnetic nanocarriers suspension under synergetic therapy, combining photothermal therapy (laser power 100 mW) and magnetic fluid hyperthermia therapy (magnetic field strength 200 Oe).

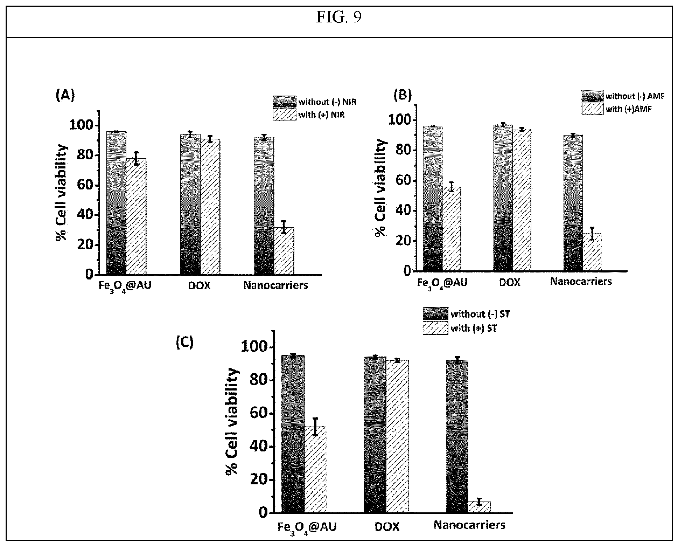

FIG. 9. *In vitro* investigation of viability of the breast cancer cell line MCF7 in the presence of nanocarries. [A] Results of photothermal therapy PTT with use of NIR laser (808 nm, 100 mW) [B] Results of magnetic hyperthermia MHT with applied alternative magnetic field (200 Oe, 265 kHJz). [C] Results synergistic therapy PTT+MHT.. Cancer cells were treated for 30 minutes respectively with p$Fe_3O_4$@Au, doxorubicin DOX and photo-magnetic nanocarriers with a concentration of 1 mg/mL. All experiments were carried out with appropriate control samples and their results are shown as deep-gray bars on each figure After the treatment, the cells were incubated under culture conditions for 24 h. The cell viability was calculated by using MTT assays, cell viability values are expressed as Mean ±SD, n = 3.

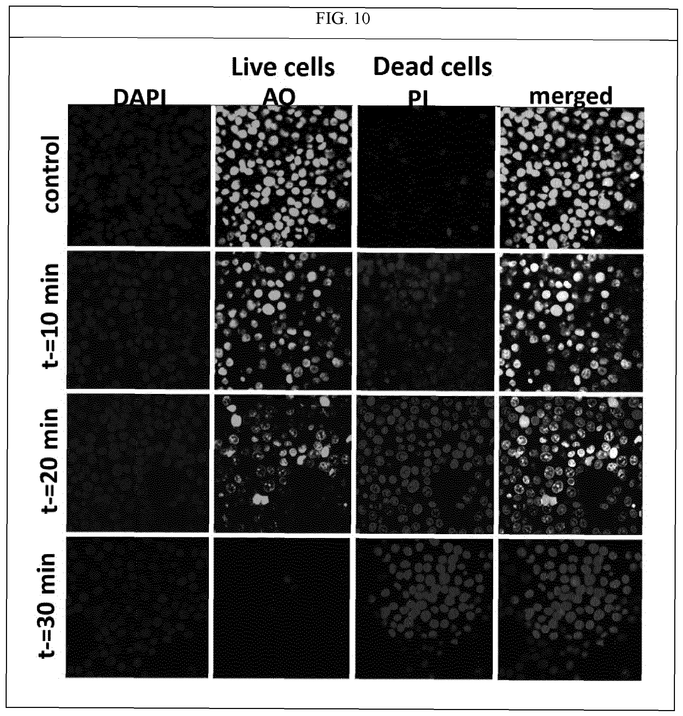

FIG. 10. Fluorescence confocal microscopic images of breast cancer cell line MCF7 untreated (control) and treated with use of synergetic therapy with photo-magnetic nanocarriers for 10, 20 and 30 min (image scale 20 μm). The cells were stained with DAPI, Acridine Orange (AO) and Propidium iodine (PI) and incubated for 24 h The background fluorescence due to DOX was removed by using LSM imaging software.

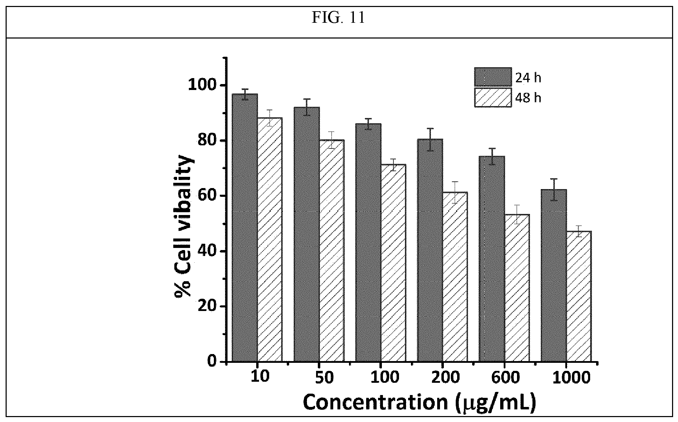

FIG. 11. Biocompatibility studies of photo-magnetic nanocarriers were perfomed with use of noncancerous cell line L929. As a measure of t biocompatibility the L929 cell viability was taken. The viability was assessed at varying concentrations of photo-magnetic nanocarriers, starting from 10 to 1000 μg/mL. Test were carried out for two different incubation times (respectively 24 and 48 h).

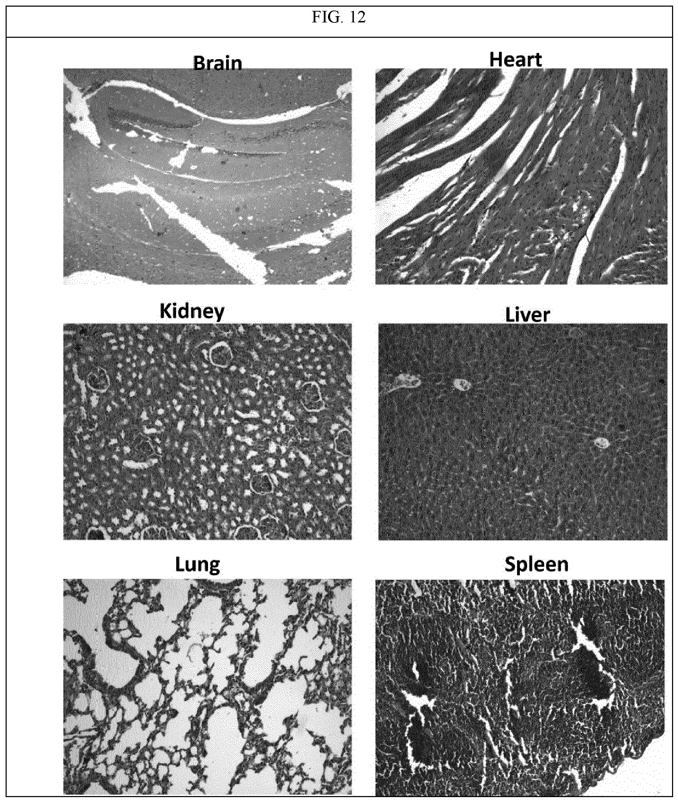

FIG. 12. *In vivo* biocompatibility studies of photo-magnetic nanocarriers. Figure shows H & E stained images of the vital organs in female swiss albino mice (liver, kidney, lungs, brain, spleen) treated with 2000 mg/kg for a 14-day period following the intraperitoneal injection of nanocarriers. The control measurements were conducted by the same treating with use of PBS buffer.

Multifunctional $Fe_3O_4$@Au core shell nanostructure functionalized with polymer and anticancer moieties having magnetic field responses and NIR absorption feature were synthesized (FIG. 1).

Several synthesis methods have been reported for $Fe_3O_4$@Au, such as the chemical co-precipitation method, hydrothermal method, high temperature synthesis method, iterative hydroxylamine seeding process, and seeded growth using citrate reduction.

In one embodiment, the invention provides a low temperature reflux method for synthesizing $Fe_3O_4$@Au having magneto-plasmonic properties. $Fe_3O_4$@Au synthesized with the method described herein has hydrodynamic size and optical properties that are optimal for nanotheranostic applications, particularly, for PTT and MHT of breast cancer.

In certain embodiments, a thin gold layer templated by cetyltrimethylammonium bromide (CTAB) was primarily deposited onto magnetic $Fe_3O_4$ layer, the overall core shell structure has 15 to 20 nm of diameter (FIG. 2). This high coverage of gold benefits not only strong light response of single nanocarrier but also support the polymer/biomolecule conjugation.

In one embodiment, the method for synthesizing $Fe_3O_4$@Au coated with polymer for example mPEG-SH, anticancer drug for example doxorubicin magneto-plasmonic nanoparticles comprises the steps of

(a) synthesis of $Fe_3O_4$@Au nanoparticles in single reaction solution using low temperature reflux method;
(b) dispersing $Fe_3O_4$@Au in a solution comprising a polymer mPEG-SH solution;
(c) formation of polymer mPEG-SH coated $Fe_3O_4$@Au nanoparticles;
(d) conjugating anticancer drug into polymer functionalized nanoparticles;
(e) grafting cancer cell targeting anticancer aptamer to the drug encapsulated nanoparticles
(f) the overall assembly of photo-magnetic nanocarrier comprising anticancer drug, aptamer, polymer conjugated magnetic-plasmonic nanoparticles

The resulting photo-magnetic nanocarriers can then be isolated using magnetic field and dispersible in water, cell media and buffer solutions.

In one embodiment, $Fe_3O_4$@Au have a magnetic responsiveness, preferably, superparamagnetic properties. The whole core shell $Fe_3O_4$@Au revealed almost zero remanence/coercivity (FIG. 3) and superparamagnetic nature as expected. The high magnetization (47.5 emu g$^{-1}$) at 300 K, enabled strong magnetic response of single nanostructure, which is desirable for MHT and magnetically targeting of cancer cells.

X-ray photoelectron spectroscopy analysis confirmed the presence of iron (Fe) oxygen (O) and gold (Au) content in the core shell nanostructure (FIG. 4). The plasmonic feature of the core shell nanostructure was subsequently studied. The plain core shell nanostructure possessed a distinct localized surface plasmon resonance (LSPR) absorption peak (FIG. 5).

In another embodiment, $Fe_3O_4$@Au have, respectively, a polymer coating on their surface, which allows for ionic binding of a therapeutic agents thereto. The clinically approved drug Doxorubicin (DOX) loading was performed on polymer functionalized (mPEG-SH@$Fe_3O_4$@Au) through EDC-NHS coupling chemistry. The interaction of DOX molecules with nanostructures was investigated using fluorescence spectroscopy. The fluorescence intensity of DOX decreased when it was conjugated on nanostructures (FIG.6A), which indicates efficient loading of DOX. Drug release was observed in water with different pH including the physiological pH of blood is 7.4 and subcellular lysosomal compartments of tumor cells pH 5.0. The drug release studies performed at pH 7.4, 5, 2 and 2. A pH-dependent DOX release profile was observed after 24 h of time (FIG 6B).

'On demand' DOX release under alternating magnetic field (AMF) is the most suitable way to achieve effective remotely active cancer therapy. Thus, the DOX release under AC magnetic field is performed in the current innovation. We could control of DOX release upon exposure of alternating magnetic field AMF (20 kAm$^{-1}$ $\approx$ 300 Oe, f= 265 kHz) in the temperature range of 37-42°C and different pH (FIG. 6C). Under 60-minute magnetic exposure we observed app. 21, 26 and 32% DOX release at pH 7.4, pH 5 and pH 2, respectively. The drug conjugated nanocarriers were exposed to magnetic field repeatedly with the magnetic field "ON" for 1 min, followed by a 10 min interval with the magnetic field being turned "OFF". The "ON/OFF" cycles were performed 6 times for 60 minute.

The photo-magnetic nanocarriers compositions, with bound to or co-loaded therapeutic agent, described herein can be used to release drugs to a subject in a controlled release fashion by applying external magnetic field. In this embodiment, photo-magnetic nanocarriers form ionic bonds with therapeutic agent and applying an alternating magnetic field (AMF) to the nanocarriers weaken the attraction and release this therapeutic agent. In a further embodiment, either an alternating magnetic field (AMF) and/or a near-infrared (NIR) light can be used to release the therapeutic cargo bound to a photo-magnetic nanocarriers.

Local ablation of tumor or hyperthermal treatment are increasingly employed for treating of many type of non resectable solid cancerous tumors, including (i) high-intensity focused ultrasound (FUS) (ii) magnetic field induced hyperthermia (MHT), (iii) radio frequency (RF) ablation, (iv) microwave ablation, and (vi) laser light ablation. These non-conventional therapeutic procedures are noninvasive, patient recovery time is shorter than the traditional surgery and do not need long hospitalization, hence recently these therapeutic modalities are taking increased attentions of both, researchers and also clinicians. Nanomedicine developments in the recent decades of twenty first century, resulted in numerous hybrid nanomaterials based theranostic probes for hyperthermal therapies. In the current innovation we showed photo-magnetic nanocarriers are ideal candidate of laser induced photothermal and magnetic field activated hyperthermia for each treatment. Furthermore, our innovation used combined strategy of photothermal therapy (PTT) and magnetic hyperthermia therapy (MHT), wand impressive induced cancer cell death is observed compared to singular modality.

The photothermal potential and temperature stability of photo-magnetic nanocarriers were characterized in cuvette phantoms simulating in water with an 808 nm NIR laser diode source. FIG. 7A depicts that the maximum change of temperature ($\Delta T$ in °C) for our nanocarriers (with very low concentration 1 mg/mL) rapidly increased with NIR irradiation power. At laser radiation exposures with power 100, 500, 750 and 1000 mW for 10 min, maximum $\Delta T$ reached respectively since 3.0 to 23.7 °C,. No significant change was observed for control water sample kept in similar conditions. The photothermal effect (PTT) of our nanocarriers was further visualized using IR thermal camera. For this measurements nanocarriers were dispersed in the water and IR-thermographic imaging was carried (FIG.7B). In the next experiments a magnetic hyperthermia (MHT) caused by our photo-magnetic nanocarriers was studied as a function of applied alternating magnetic field) strength, at fixed frequency of 265 kHz (FIG. 7C). The magnetic hyperthermia performance (change of temperature $\Delta T$ in °C) for the same nanocarriers suspension (concentration 1 mg/mL) was increasing with the magnetic field strength (100, 200 and 300 Oe).. The optimum hyperthermic temperature' treshold proposed for animal or human i.e. 42- 44 °C wass achieved by samples in all applied alternating magnetic field strengths.

For thhe complete *in vivo* tumor tissues destruction, combined therapeutic approach is particularly promising. Due to the light absorption limitation of human body PTT is less effective than MHT in vivo, while the opposite PTT is more effective than MHT *in vitro.* Thus, the synergies of the two therapies brought the best results both *in vitro* and *in vivo.* FIG. 8 shows the results of the combined therapy (PTT + MHT) and we found that within low laser (NIR) power (100 mW) and AMF field strength (200 Oe), hyperthermic effect is observed. Thus the combination of PTT and MHT, examined on the cellular level, mainly rely on plasmonic and magnetic properties of nanocarriers (FIG. 9). For instance, the viability of the breast cancer cell line MCF7 in presence of photo-magnetic nanocarriers and NIR irradiation is about 32 % (only PTT) (FIG. 9A), in presence of alternating magnetic field (only MHT) is about 25 % (FIG. 9B), and only 4 % in case of joined therapy (PTT+MHT) (FIG. 9C). This result shows the synergism of dual treatment. In order to validate the effectiveness of of synergetic therapy, the cancerous cells line MCF7, were subjected to combined therapy with photomagnetic nanocarries for 30 minutes. For cell survival assesement the cells were stained using DAPI (visualizes all cells), acridine orange AO (visualizes live cells)), propidium iodine PI (visualizes death cells) and their number was observed by use of fluorescence microscopy (FIG. 10). It was observed that the percentage of dead cells was increasing linearly throughout the combination therapy, and after 30 minutes of this dual therapy, almost all cells were death.

The cell viability analysis of non-cancerous L929 cells treated with $Fe_3O_4$@Au formulation, show obviously that the cell viability decreased as the nanoparticles concentration increased. However, respectively 62.5% and 47.2% cells variability was observed even for the higher concentration (1000 $\mu$g/mL) after 24 h and 48 h of treatment (FIG. 11), respectively. As it is known that multifunctional nanostructures are highly effective but also toxic to vital organs, we performed a preliminary in vivo cytotoxicity and histopathological examination of exposed mice organs focusing on tissue damage, inflammation or lesions (FIG. 12). Nanostructures were administrated to the organisms via intravenous route (i.v.). The *in vivo* study showed no indication of serious injuriousness or toxicity. It was shown as well that the safe limit of therapeutically effective dose of $Fe_3O_4$@Au formulation is up to 2000 mg/kg.

Following are examples which illustrate procedures for practicing the invention. The following examples, including the experiments conducted and results achieved are provided for illustrative purposes only and are not to be construed as limiting upon the present invention.

Example 1-Synthesis of $Fe_3O_4$@Au core shell nanoparticles

Core shell $Fe_3O_4$@Au nanoparticles having magnetic and plasmonic were synthesized by the low temperature chemical reflux approach. Next, using with the same method, the nanoparticles of $Au^{3+}$ have been attached on the surface of magnetic $Fe_3O_4$ in the same solution. After this step, dispersible core-shell magnetic and plasmonic active nanoparticles have been formed using CTAB capping procedure. The narrow size distribution of core shell assembly, in between 15 to 20 nm, is the key for monodispersity of $Fe_3O_4$@Au.

After first layer formulation of Au shell on the surface of $Fe_3O_4$, additional layer of gold is developed using sodium citrate and ascorbic acid reducing chemistry. Additional two layers of gold develope to form of the core shell structure. After the final step of gold capping, dark brown initial solution is transformed into red/pink color. With increasing the amount of Au, significant absorbance appeared at the wavelength between 460 nm and 600 nm (FIG. 5A).

Example 2-Synthesis of photo-magnetic nanocarriers

This example illustrates the synthesis of theranostics nanocarriers with anticancer and targeting and photo-magnetic properties. The photo-magnetic nanocarriers are developed using step by step chemical process. In the first step, $Fe_3O_4$@Au prepared as above have CTAB on their surface. This CTAB molecules are removed or exchanged with polymer for example thiolated polyethylene glycol (mPEG-SH) molecules. 0.5 to 1.0 M mPEG-SH solution in water is added into 10-20 mL CTAB grafted $Fe_3O_4$@Au nanoparticles solution in double distilled water. The pH of the total solution was adjusted to 8 with additional 12 h magnetic stirring. The resultant solution then washed by water and centrifuged. The mPEG-SH grafting is changing the surface charge of nanoparticles and these was observed by significant decrease in $\zeta$-potential of CTAB grafted $Fe_3O_4$@Au from +30 to +6 of mPEG-SH@$Fe_3O_4$@Au.

Anticancer drug for example, doxorubicin is further conjugated into above assembly using the EDC (1-ethyl-3-(3-dimethylaminopropyl)carbodiimide hydrochloride) and NHS (Sulfo-NHS (N-hydroxysulfosuccinimide)) coupling chemistry methodology. The mPEG-SH encapsulated $Fe_3O_4$@Au nanoparticles from the above method (10 mg) were dissolved in 2 mL of DI water and EDC-NHS solution, and magnetically stirred for 12 h. The average particle size was measured as a hydrodynamic radius by dynamic light scattering (DLS) and surface charge was measured using zeta potential techniques. In the final step, DOX conjugated nanoparticles (5 to 10 mg) are added into 5 mL conjugation buffer (100 mM phosphate buffer solution, 150 mM sodium chloride, 2.5 mM ethylene-diamine-tetraacetic acid, pH 7.2) containing 100 to 500 nmol AS1411 aptamer with stirring for additional 2 h. The final resulting product is denoted as photo-magnetic nanocarriers.

Example 3- Multimodal nanotheranostics of photo-magnetic nanocarriers

Photothermal therapy (PTT) and magnetic hyperthermia therapy (MHT) were conducted to demonstrate multimodality of photo-magnetic nanocarriers. FIG. 7A and 7C show the results of PTT and MHT studies in case of photomagnetic nanocarriers suspensions with concentration 1 mg/mL. PTT study was performed using of NIR laser with wavelength 808 nm as a function of its power (respectively 100,500, 750 and 1000 mW), while MHT study was performed as function of applied magnetic field strength AMF (respectively 100, 200 and 300 Oe) The correlation between laser power and temperature increase as well as applied magnetic field strength and temperature increase was observed. Both, PTT and MHT has caused the temperature rise of the solutions, thus the thermal effect was confirmed..

The effectiveness of multimodal combination therapy and its synergistic effect was further validated on breast cancer cell line MCF7 (FIG. 9A-C). In case of dual therapy (PTT + MHT) almost 95- 96% breast cancer cells died and only 5 to 4% cells were viable after 24 h of the dual therapy (FIG. 9C). This result shows the effectiveness of multimodal combination therapy with use of our nanocarriers. Also this strong anticancer therapeutic effect of PTT+MHT dual therapy using photo-magnetic nanocarriers suggest that these nanocarriers can be successfully used as multi-modal therapeutic agents for breast and other cancers.

Advantages are presented below.

➢ Integrating multiple discrete functionalities into core shell photo-magnetic architecture with distinctive magnetic/plasmonic properties.

➢ Photo-magnetic nanostrtcures have multifunctionality of drug carriers with complementary theranostic modalities.

➢ In this innovation, the incorporation of gold shell on spherical magnetic core is achieved via low temperature chemical reflux method. The core shell structure, which was intrinsically different from previous direct chemical or heterogeneous conjugation of the two differnt components.

➢ The magnetic core consisted of stacked primary iron oxide ($Fe_3O_4$) nanocrystals yields strong superparamagnetic response with excellent permeability for magnetically actuated drug delivery.

➢ The highly preserved magnetic and plasmonic feature of nanostructures enabled magneticaly active drug release and photo-magnetic hyperthermia capabilities.

➢ Multifunctionality of the nanocarriers enabled pH/local heating dual-responsive drug release with minor leakage at neutral pH.

➢ Under the magnetic hyperthermia using the nanocarriers, the magnetothermal-chemo synergistic therapy was highly efficient on breast cancer cells eradication.

➢ The safety limit of Fe304@Au formulation is up the dosage of 2000 mg /kg.

Material and methods

Material

Fe(III)acetylacetonate MW= 353.17 (97% Sigma Aldrich), Oleylamine technical grade, 70% (Sigma Aldrich), Oleic acid ≥99% (GC) (Sigma Aldrich), Gold(III) chloride hydrate, $HAuCl_4$ $3H_2O$ 99.995% trace metals basis (Sigma Aldrich), exadecyltrimethylammonium bromide (CTAB) BioXtra, ≥99% (Sigma Aldrich), L-Ascorbic acid 99% (Sigma Aldrich) methoxy polyethylene glycol thiol (mPEG-SH), NaOH, PBS buffer, glutaraldehyde, ethanol, fluorescein diacetate (FDA), propidium iodide (PI). The thiolated DNA aptamer (AS1411) and its complementary DNA labeled with 5'-FAM were obtained from Integrated DNA Technologies, BVBA. The aptamer sequences are listed as follows: C6-TTGGT-GGTGGTTGTGGTGGTGGTGG-3',

*Synthesis of photo-magnetic nanocarriers*

Synthesis procedure of photo-magnetic nanocarriers involves four intermediate steps these are: 1. Synthesis of 15 nm $Fe_3O_4@Au$ nanoparticles, 2. Ligand xxchange on CTAB-Capped $Fe_3O_4@Au$ nanoparticles with mPEG-SH, 3. Model anticancer drug Doxorubicon (DOX) conjugation on mPEG-SH functionalized $Fe_3O_4@Au$ and 4. Adding anticancer aptamer AS1411 to DOX @mPEG-SH functionalized $Fe_3O_4@Au$.

1. Synthesis of $Fe_3C_4@Au$ nanoparticles

~15 to 20 nm in gold shell with magnetic core nanoparticles with plasmonic and properties were prepared by following a four step chemical reflux method.

*First step*

0.1 to 0.5M Fe(III) dissolved in 9 to 45 mL oleylamine and 5 to 25 ml of oleic acid, then 25 to 100 mg Au (III) is dissolved in the 1 to 5 mL oleylamine and the total solution of 15 to 75 mL is kept on stirring for 30 min at 80 to 90°C. Then the solution is refluxed for 30 min at about 325 to 350°C. The prepared nanoparticles are washed with chloroform and ethanol and centrifuged for 10 to 20 min at 4500 rpm.

*Second step*

Prepared $Fe_3O_4@Au$ nanoparticles are redispersed in 9 to 45 mL of chloroform. 25 to 100 mg Au (III) is dissolved in 1 to 5 mL oleylamine and added into chloroform solution with further 24 h stirring at 25 to 30°C. Then the solution is centrifuged for for 20 to 30 min at 4400 rpm and washed with hexane and alcohol.

*Third step*

In the third step prepared $Fe_3O_4@Au$ nanoparticles are dispersed in 3 to 15 mL of chloroform and this solution added dropwise into solution containing 30 to 50 ml DI water with 100 to 500 mg CTAB and 20-50 mg citrate solution with stirring for 30 to 40 min at 100°C. finally water dispersed $Fe_3O_4@Au$ nanoparticles centrifuge 4500 rpm 10 to 20 min.

*Fourth step*

In the final step, 100 mg CTAB is dissolved in 20 ml DI water and the prepared $Fe_3O_4@Au$ nanoparticles are added in to this solution and sonicated for 2 min. In the other step solution of 200 mg CTAB, 20 mg ascorbic acid and 25 mg $HAuCl_4$ is prepared in 10 mL DI water. Both solution mixed during sonication and keep it on stirrer for 24 h at 40°C. Finally centrifuged at 4400 rpm and washed DI water and dried at room temperature.

2. Ligand exchange with mPEG-SH:

The as prepared $Fe_3O_4@Au$ nanoparticles are containing CTAB on their surface. These CTAB molecules are is exchanged with mPEG-SH (MW = 750 Da). In brief, CTAB grafted $Fe_3O_4@Au$ nanoparticles are washed with water 2 to 3 times and centrifuged at 5000 rpm to remove excess CTAB. 0.5 to 1.0 M mPEG-SH was added to the 10 to 20 mL $Fe_3O_4@Au$ nanoparticles solution in DI water. The pH of the total solution was adjusted to 8 by adding NaOH and the solution further kept on stirring for 12 h. The resultant solution then washed by water and centrifuged at 5000 rcf. for 10 to 15 min. The mPEG-SH grafting was observed by significant decrease in $\zeta$-potential.

*3. Doxorubicin conjugation:*

To encapsulate DOX into mPEG-SH functionalized $Fe_3O_4$@Au nanoparticles EDC/NHS coupling chemistry is used. Initially 1 to 2 mg DOX is dissolved in 1 to 2 mL DI water, to this solution EDC (1 to 2 mg,) in 2 to 3 mL of $H_2O$, NHS (2 to 4 mg,) was added drop wise and solution kept overnight on magnetic stirrer. Further the prepared solution is added into 10 to 20 mg mPEG-SH functionalized $Fe_3O_4$@Au nanoparticles solution made in 10 to 15 mL water and final solution is kept for more 12 h on stirring. All DOX conjugation experiments are carried in dark conditions. The DOX encapsulated particles were washed thoroughly with milli-pore $H_2O$. In order to characterize the doxorubicin loading directly on nanoparticle surface, the particles were dispersed in 1 ml of DI water (0.5 mg mL$^{-1}$) and UV absorbance measurements were performed on the hybrid suspension. All the absorbance measurements were performed using a Cary 60 UV-vis spectrophotometer (Agilent Technologies, Inc.,USA). The doxorubicin was quantified by measuring the absorbance at 480 nm. Fluorescence measurements on free DOX and conjugated DOX (Ex. 480 nm, Em. 590 nm) were performed using a Fluorolog-3 spectrofluorometer to calculate the loading efficiency. The loading efficiency (w/w%) was calculated using the following equation

$$\%loading\ \text{efficiency} = \frac{I_{DOX} - I_S - I_W}{I_{DOX}} \times 100 \tag{1}$$

where, $I_{DOX}$ is the fluorescence intensity of only DOX solution, $I_S$ the fluorescence intensity of DOX conjugated nanoparticles and $I_W$ the fluorescence intensity of washed DOX (physically adsorbed DOX molecules). The release behaviour of DOX from the nanoparticles was investigated in water with different pH values. Briefly, nanoparticle samples with different pH (2 to 7.4) were diluted in water and mounted into a shaking bed at 37 °C with a rotation speed of 150 rpm. To monitor the release behaviour, samples were periodically taken out at desired time intervals and subjected to magnetic decantation. The DOX concentration in the supernatant was analysed using a UV/vis spectrophotometer and spectrofluorometer, parallel. The concentration of DOX is estimated by a standard DOX concentration curve generated from a series of DOX solutions with various concentrations. The amount of DOX released from the nanoparticles was the amount of DOX in the supernatant detected by UV-vis spectrometer.

*4. Aptamer functionalization:*

The DOX conjugated $Fe_3O_4$@Au nanoparticles (5 to 10 mg) are added into 5 to 20 mL conjugation buffer (100 mM PBS, 150 mM sodium chloride, 2.5 mM EDTA, pH 7.2) containing 100 to 500 nmol AS1411 aptamer with stirring 2 to 4 h. The final resulting product is denoted as photo-magnetic nanocarriers.

*Photo-magnetic PTT treatment on cancer cells:* The initial $2 \times 10^5$ cells/mL cell number was maintained for all PTT experiments. The PTT treatment was applied to either free cells (without nanocarriers) and with all other controls in media, to estimate the nanocarriers mediated PTT killing in cellular conditions. Photothermal conversion efficacy of nanocarriers was analyzed with an 800-nm infrared diode laser with a varying power density of 0.1 to 1 W/cm$^2$, The PTT LASER device is built in the in-house laboratory.

*In vitro* hyperthermia on cancer cells

MCF7 cells were mixed with the nanocarriers with a 1 mg mL$^{-1}$ concentration to evaluate *in-vitro* hyperthermia performance. During the procedure, AC magnetic fields remained switched on until the upper hyperthermia temperature limit of 43-44 °C was attained. All *in-vitro* hyperthermia studies were performed at applied frequency of 265 kHz and the AC magnetic field (AMF) was kept at 300 Oe. The media containing nanocarriers suspended with 1 mL cancer cells ($2\times10^5$ cells/mL) was placed at the center of the coil. The samples containing NPs and cells were heated for 60 min and the temperature of the system was maintained at 43-44 °C during whole experiment. Immediately after exposure, cells were placed in 96 well plates in triplicates and incubated for 24 h. The cell viability was measured using MTT assay while, simultaneously, the cell samples were incubated in slide petri dishes containing 2 mL DMEM for further confocal microscopy imaging after FITC, PI and DAPI staining.

*Photo-magnetic nanocarriers characterization*

Magnetic characterization of powder samples and colloidal suspensions are performed in a Quantum Design MPMS-5S SQUID magnetometer. The particle size and shape was determined by transmission electron microscopy (TEM, Philips CM200 model, operating voltage 20-200kV, resolution 2.4 Å). Magnetic hyperthermia of nanocarriers for hyperthermia application was performed in plastic microcentrifuge tube (1.5 mL) by using an induction heating unit (Easy Heat 8310, Ambrell; UK) with a 6 cm diameter (4 turns) heating coil. A provision of water circulation in the coil was made to maintain an ambient temperature. The water-suspended nanocarriers (1 mL) were placed at the center of the coil, and 265 kHz AC current was applied. The particles with different concentrations were ultrasonicated for 5 min to disperse in the carrier fluid. Samples were heated for 10 min with the desired current (200 to 600 A that can generate magnetic field of 83.8 to 502.8 Oe, respectively). Temperature was measured using optical fiber probe with an accuracy of $\pm 0.1\,°C$.

*In vivo biocompatibility and bio-distribution*

*In vivo* (animal experiments) study were conducted by prior approval from the institutional animal ethics committee of Manipal University, Manipal, India and in accordance with guidelines mentioned by Ministry of Environment and Forest, Govt. of India. Briefly, female Swiss Albino mice of 28-35 g were selected for the study. Animals were provided by the central animal facility from the institute and acclimatized under natural light/dark conditions for 2 weeks at temperature of $25 \pm 2\,°C$, RH 50-60% before experiments. All animals were divided into group I and II, each containing three animals. Group I is the control group, treated with normal saline, group II was treated with three different concentrations of nanocarriers (2000 mg/kg) intravenously through the tail vein with prior calculation of dose as per body weight. After experimentation animals were sacrificed by cervical dislocation and tissues (hearts, lungs, liver, kidney, spleen, and brain) from all experimental animal groups were stored in 10% buffered formalin. Collected tissues were embedded in paraffin blocks and sliced into 5 $\mu$m in thickness, mounted on slides, stained with hematoxylin and eosin, and examined under light microscope (Olympus BX-50 Corporation Tokyo, Japan) to get histopathological images.

**Claims**

1. Biologically active nanoparticles with a magnetic core and a gold coating **characterized in that** they have magnetic and plasmonic properties activated by an alternating magnetic field and/or infrared light, and additionally the nanoparticles are in the form of a core-shell nanoparticle where the magnetic core is $Fe_3O_4$ and the plasmonic surface is at least one gold layer.

2. Nanoparticles according to claim 1, wherein the nanoparticles are obtained by the method of low temperature chemical reflux.

3. Nanoparticles according to claim 1, wherein the Au3 + are attached to the magnetic $Fe_3O_4$ surface obtained using the hexadecyltrimethylammonium bromide CTAB capping procedure.

4. Nanoparticles according to claim 1 or 2 or 3, wherein the size of the core-shell structure is between 15 and 20 nm.

5. Nanoparticles according to claims 1-4, wherein the nanoparticles are additionally conjugated by ionic bonding to a biologically active agent and/or a drug.

6. Nanoparticles according to claim 5, wherein the biologically active agent is an aptamer.

7. Nanoparticles according to claim 5, wherein the drug is a cytostatic drug.

8. Nanoparticles as described in claims 1-7 for use in the treatment of tumours in an alternating magnetic field and/or under the influence of near-infrared light.

9. Nanoparticles as described in claims 1-7 for use as a diagnostic agent for tumours visualization in computed tomography and/or magnetic resonance imaging and/or Raman spectroscopy.

10. Nanoparticles as described in claims 1-7 for use in the treatment of cancers in an alternating magnetic field and/or under the influence of near-infrared light and diagnosis of cancers by computed tomography and/or magnetic resonance imaging and/or Raman spectroscopy.

11. Nanoparticles as described in claims 1-7 for use as a carrier for drugs and/or biologically active agents.

12. Method for obtaining nanoparticles described in claims 1-7, wherein nanoparticles are synthesized by low temperature chemical reflux.

13. The method according to claim 12, wherein nanoparticles are obtained using the method of low temperature chemical reflux, wherein Au3+nanoparticles nanoparticles are bonded to the magnetic $Fe_3O_4$ surface, and then hexadecylt-rimethylammonium bromide CTAB is attached using a capping procedure.

14. Method according to claims 12-13, wherein CTAB molecules are removed and replaced with a polymer, preferably thiolated polyethylene glycol (mPEG-SH) molecules, and then the nanoparticle is coupled with a biologically active agent and/or a drug using 1-ethyl-3-(3-dimethylaminopropyl)carbodiimide hydrochloride EDC and sulfo-NHS (N-hydroxysulfosuccinimide).

15. Method according to claim 14, wherein nanoparticles are conjugated with the drug in the form of a cytostatic.

16. Method according to claim 14, wherein nanoparticles are conjugated to the active agent in the form of aptamer by addition into conjugation buffer containing aptamer.

FIG. 1

**Photo-Magnetic Nanocarrier**

Plasmonic shell

Magnetic core

Polymer ligand

Anticancer drug

Cancer cell targeting aptamer

**Core: Superparmagnetic, T₂ MR imaging**

**Gold layer: Light active, Raman spectroscopy & CT imaging**

FIG. 2

FIG. 3

FIG. 4

FIG. 5

FIG. 6

FIG. 7

FIG. 8

FIG. 9

FIG. 10

FIG. 11

## FIG. 12

**Brain**

**Heart**

**Kidney**

**Liver**

**Lung**

**Spleen**

Europäisches
Patentamt

European
Patent Office

Office européen
des brevets

## EUROPEAN SEARCH REPORT

Application Number

EP 20 19 2433

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FARUQ MOHAMMAD ET AL: "Influence of Gold Nanoshell on Hyperthermia of Superparamagnetic Iron Oxide Nanoparticles", JOURNAL OF PHYSICAL CHEMISTRY C, vol. 114, no. 45, 18 November 2010 (2010-11-18), pages 19194-19201, XP055029804, ISSN: 1932-7447, DOI: 10.1021/jp105807r * Abstract; pages 19199-19200 and Figure 8 * | 1-3, 8-11, 14-16 | INV. A61K41/00 A61P35/00 |
| X | RUDAKOVSKAYA P G ET AL: "Synthesis of magnetite-gold nanoparticles with core-shell structure", MOSCOW UNIVERSITY CHEMISTRY BULLETIN, ALLERTON PRESS, HEIDELBERG, vol. 70, no. 3, 4 August 2015 (2015-08-04), pages 149-156, XP035524329, ISSN: 0027-1314, DOI: 10.3103/S0027131415030104 [retrieved on 2015-08-04] * page 150, right column, penultimate paragraph; page 151; page 154 * | 1-4,8-16 | |
| X | LI ZHIWEI ET AL: "Magnetic Targeting Enhanced Theranostic Strategy Based on Multimodal Imaging for Selective Ablation of Cancer", ADVANCED FUNCTIONAL MATERIALS, vol. 24, no. 16, 5 December 2013 (2013-12-05), pages 2312-2321, XP055772371, DE ISSN: 1616-301X, DOI: 10.1002/adfm.201303345 * Abstract; page 2313, right column, penultimate paragraph and figure 2 * | 1-3, 8-11, 14-16 | TECHNICAL FIELDS SEARCHED (IPC) A61K |

-/--

The present search report has been drawn up for all claims

| Place of search | Date of completion of the search | Examiner |
|---|---|---|
| The Hague | 4 February 2021 | Burema, Shiri |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

## EUROPEAN SEARCH REPORT

Application Number

EP 20 19 2433

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RAMOS-TEJADA MARÍA DEL MAR ET AL: "Preparation of multi-functionalized Fe304/Au nanoparticles for medical purposes", COLLOIDS AND SURFACES B: BIOINTERFACES, vol. 128, 1 April 2015 (2015-04-01), pages 1-7, XP055772368, NL ISSN: 0927-7765, DOI: 10.1016/j.colsurfb.2015.02.002 * Abstract; paragraph 3.3 * | 1-3,5-7, 11,14-16 | |
| X | MOHAMMAD FARUQ ET AL: "Doxorubicin-loaded magnetic gold nanoshells for a combination therapy of hyperthermia and drug delivery", JOURNAL OF COLLOID AND INTERFACE SCIENCE, ACADEMIC PRESS,INC, US, vol. 434, 2 August 2014 (2014-08-02), pages 89-97, XP029063575, ISSN: 0021-9797, DOI: 10.1016/J.JCIS.2014.07.025 * paragraph 3.1; Abstract, figure 1, page 90, right column * | 1-3, 5-11, 14-16 | |
| X | Venugopal Indu: "Magnetic field-enhanced cellular uptake of doxorubicin loaded magnetic nanoparticles for tumor treatment", , 1 January 2016 (2016-01-01), XP055772416, Retrieved from the Internet: URL:https://iopscience.iop.org/article/10.1088/2053-1591/3/9/095010/ampdf [retrieved on 2021-02-04] * Abstract; paragraph 4.1; pages 17-18 * | 1-8,10, 11,14-16 | |

TECHNICAL FIELDS SEARCHED (IPC)

-/--

The present search report has been drawn up for all claims

| Place of search | Date of completion of the search | Examiner |
|---|---|---|
| The Hague | 4 February 2021 | Burema, Shiri |

EPO FORM 1503 03.82 (P04C01)

Europäisches
Patentamt

European
Patent Office

Office européen
des brevets

## EUROPEAN SEARCH REPORT

Application Number

EP 20 19 2433

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Danafar Hossein ET AL: "Co -delivery of Sulforaphane and Curcumin with PEGylated Iron Oxide- Gold Core Shell Nanoparticles for Delivery to Breast Cancer Cell Line", , 1 January 2018 (2018-01-01), XP055772419, Retrieved from the Internet: URL:http://ijpr.sbmu.ac.ir/article_2239_0e 937055e4e02925e4329f8d1eb549b9.pdf [retrieved on 2021-02-04] * Abstract and figure 1 * ----- | 1-3,5-7, 11,14-16 | |
| X | BINAYMOTLAGH ROYA ET AL: "Selective chemotherapy and imaging of colorectal and breast cancer cells by a modified MUC-1 aptamer conjugated to a poly(ethylene glycol)-dimethacrylate coated $Fe_3O_4$ -AuNCs nanocomposite", NEW JOURNAL OF CHEMISTRY, vol. 43, no. 1, 1 January 2019 (2019-01-01), pages 238-248, XP055772593, GB ISSN: 1144-0546, DOI: 10.1039/C8NJ04236E Retrieved from the Internet: URL:https://pubs.rsc.org/en/content/articl epdf/2019/nj/c8nj04236e> * Abstract, conclusion and figure 1 * ----- -/-- | 1-3, 5-11, 14-16 | |

TECHNICAL FIELDS
SEARCHED     (IPC)

The present search report has been drawn up for all claims

| Place of search | Date of completion of the search | Examiner |
|---|---|---|
| The Hague | 4 February 2021 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EUROPEAN SEARCH REPORT

Application Number

EP 20 19 2433

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BANESHI MARZIEH ET AL: "A novel theranostic system of AS1411 aptamer-functionalized albumin nanoparticles loaded on iron oxide and gold nanoparticles for doxorubicin delivery", INTERNATIONAL JOURNAL OF PHARMACEUTICS, vol. 564, 10 June 2019 (2019-06-10), pages 145-152, XP085689544, ISSN: 0378-5173, DOI: 10.1016/J.IJPHARM.2019.04.025 * Abstract * ----- | 1-16 | |

TECHNICAL FIELDS SEARCHED (IPC)

The present search report has been drawn up for all claims

| Place of search | Date of completion of the search | Examiner |
|---|---|---|
| The Hague | 4 February 2021 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017191219 A
- WO 2016073348 A

- WO 2015023653 A


**Non-patent literature cited in the description**

- **ESPINOSA A ; DI CORATO R ; KOLOSNJAJ-TABI J ; FLAUD P ; PELLEGRINO T ; WILHELM C.** Duality of Iron Oxide Nanoparticles in Cancer Therapy: Amplification of Heating Efficiency by Magnetic Hyperthermia and Photothermal Bimodal Treatment. *ACS Nano,* 2016, vol. 10, 2436-46
- **HUANG L ; AO L ; HU D ; WANG W ; SHENG Z ; SU W.** Magneto-Plasmonic Nanocapsules for Multimodal-Imaging and Magnetically Guided Combination Cancer Therapy. *Chem Mater,* 2016, vol. 28, 5896-904, https://doi.org/10.1021/acs.chemmater.6b02413
- **ESPINOSA A ; BUGNET M ; RADTKE G ; NEVEU S ; BOTTON GA ; WILHELM C et al.** Can magneto-plasmonic nanohybrids efficiently combine photothermia with magnetic hyperthermia?. *Nanoscale,* 2015, vol. 7, 18872-7, https://doi.org/10.1039/c5nr06168g

- **TSENG Y-J ; CHOU S-W ; SHYUE J-J ; LIN S-Y ; HSIAO J-K ; CHOU P-T.** A Versatile Theranostic Delivery Platform Integrating Magnetic Resonance Imaging/Computed Tomography, pH/ cis -Diol Controlled Release, and Targeted Therapy. *ACS Nano,* 2016, vol. 10, 5809-22, https://doi.org/10.1021/acsnano.5b08130
- **THORAT ND ; BOHARA RA ; NOOR MR ; DHAMECHA D ; SOULIMANE T ; TOFAIL SAM.** Effective Cancer Theranostics with Polymer Encapsulated Superparamagnetic Nanoparticles: Combined Effects of Magnetic Hyperthermia and Controlled Drug Release. *ACS Biomater Sci Eng,* 2017, vol. 3, 1332-40, https://doi.org/10.1021/acsbiomaterials.6b00420